## Europäisches Patentamt
## European Patent Office
## Office européen des brevets

(11) Publication number: **0 039 136**
**B1**

(12) **EUROPEAN PATENT SPECIFICATION**

(45) Date of publication of patent specification: **04.12.85**

(51) Int. Cl.⁴: **G 01 N 27/28, G 01 N 27/46**

(21) Application number: **81301299.4**

(22) Date of filing: **26.03.81**

(54) **Electrode device, method for its preparation, and dispersion useful in the method.**

(30) Priority: **27.03.80 DK 1332/80**

(43) Date of publication of application:
**04.11.81 Bulletin 81/44**

(45) Publication of the grant of the patent:
**04.12.85 Bulletin 85/49**

(84) Designated Contracting States:
**AT BE CH DE FR GB IT LI LU NL SE**

(56) References cited:
**DE-A-1 523 068**
**DE-A-1 623 064**
**DE-A-2 023 584**
**GB-A-1 200 595**
**US-A-4 198 280**

(73) Proprietor: **RADIOMETER A/S**
**Emdrupvej 72**
**DK-2400 Copenhagen NV (DK)**

(72) Inventor: **Poulsen, Jorgen**
**Bissensvej 3**
**DK-7000 Fredericia (DK)**

(74) Representative: **Boon, Graham Anthony et al**
**Elkington and Fife High Holborn House 52/54**
**High Holborn**
**London, WC1V 6SH (GB)**

Courier Press, Leamington Spa, England.

## Description

The present invention relates to an electrode device for electrochemical measurement of the concentration or the partial pressure of a gas in a liquid or a gas mixture. The electrode device is of the kind which comprises a measuring surface constituted by or comprising the sensitive part of a measuring electrode and a membrane defining a volume of an electrolyte solution (in the following designated "electrolyte" for brevity) which is in contact with the measuring electrode; said membrane being placed in front of the measuring surface and being permeable to the gas which is to be determined. In such electrode devices, the gas diffuses through the membrane and is detected either directly at the measuring electrode such as is the case in the polarographic determination of the partial pressure of oxygen or reacts with the electrolyte to form a product which is detected such as is the case, e.g., in the potentiometric determination of the partial pressure of carbon dioxide or ammonia by means of a pH-sensitive measuring electrode.

The electrolyte layer which is placed between the permeable membrane and the measuring electrode is decisive to the function of the electrode device. If the electrolyte layer is thin, a desirably short response time of the electrode device is obtained, but at the same time there is a risk that the electrode device dries, if this device is not kept constantly moistened and a risk that due to evaporation of water a noticeable change of the electrolyte concentration may take place and thereby a drift of the measuring result of the electrode device. If the electrolyte layer is thick, the equilibration will become slow, and the response time will, therefore, be long, especially when the partial gas pressure to be measured is low.

Hence, in order to obtain a quick, uniform response time of the electrode device and to obtain reproducible measuring results, it is necessary that the electrolyte layer present between the membrane and the measuring electrode is well defined and, especially in view of the response time, as thin as possible. In the mutual arrangement of the measuring electrode and the membrane, care should be taken that these do not touch each other as such a direct contact between the sensitive part of the measuring electrode and the permeable membrane, may make it impossible to obtain accurate and reproducible measurements.

In the known electrode devices of the above-mentioned type, the thickness of the electrolyte layer is determined by a distance keeping means, a so-called "spacer", placed between the measuring electrode and the membrane. Thus, German Offenlegungsschrift No. 25 39 771 discloses spacers of film of cellophane or cuprophane, a mesh or a microporous polypropylene film, and German Auslegeschrift No. 16 23 064 discloses the insertion of an electrolyte-saturated film of for example cellophane or an electrolyte-saturated sheet of cellulose paper between the measuring electrode and the membrane in a membrane-covered electrode, and also states that a mesh having a thickness of 0.1 mm and a large pore volume may be inserted.

By using spacers of paper, it is possible to provide an electrolyte layer having a thickness of about 20 microns when the paper is, e.g. hard, porous, non-compressible, electrochemically inactive Joseph paper, and with cuprophane film, which, however, is compressible, a liquid layer of the same order of magnitude may be obtained. When using mesh of, e.g. polyethylene terephthalate, a liquid layer thinner than of the order of magnitude of 50 microns is hardly obtainable, and this is often too much as the response time of the electrode then will be too long.

The present invention provides an electrode device which offers new possibilities for obtaining an effective adjustment of the thickness of the electrolyte layer in a satisfactory and practical manner and which makes it possible to maintain thinner electrolyte layers than hitherto.

According to the present invention there is provided an electrode device for electrochemical measurement of the concentration or the partial pressure of a gas in a liquid or a gas mixture, comprising a measuring surface constituted by or comprising the sensitive part of a measuring electrode and a membrane which is placed in front of the measuring surface and limits a volume of an electrolyte solution which is in contact with the measuring electrode, said membrane being permeable to the gas species to be determined, characterized in that the electrode device comprises particles dispersed substantially in a monolayer in the electrolyte solution between the measuring surface and the membrane in a sufficient amount to keep the membrane in a distance from the measuring surface, the monolayer making contact with both the measuring surface and the membrane, the particles having such small compressibility that they do not undergo any substantial change of dimensions at pressures occurring during storage, application and handling of the electrode device.

When utilizing such a particle system to maintain the distance between the measuring surface and the membrane in accordance with the principles of the present invention, it is, firstly, possible to establish very small well-defined distances which could not have been established utilizing any known spacer, with the consequent advantages with respect to electrode response and adaption of the electrode device for special purposes, and, secondly, a far more simple handling is obtained as will appear from the following.

A sufficiently well-defined distance between the membrane and the measuring surface is obtained, by the particles being, as already stated, substantially in a monolayer, i.e. there is essentially at no place throughout the measuring surface more than one layer of particles between the measuring surface and the membrane, that is to say, there are no lumps or "towers" of particles which at any place establish a greater distance between the measuring surface and the membrane than the one given by the size of the individual particles. It has been found that the monolayer does not have to be a "dense" monolayer (a "dense" monolayer of, e.g. spherical particles will correspond to dense packing of the particles in the monolayer).

In connection with the particles having a small compressibility, it is important that the particles are essentially non-plastic, i.e. that they do not, in the course of time, undergo any change of dimensions under the constant influence of pressure exerted by the membrane. In principle, any material suitable for the purpose of the present invention will be a material which to a greater or lesser extent shows elasticity so that the particles, after mounting of the membrane, are in an equilibrium deformation. It is evident that it is most suitable when the elastic deformation of the particles is small.

The particles to be used according to the present invention are preferably of a size of between 2 and 50 microns, especially of a size between 2 and 30 microns, as experiments have shown that particles of this size give an excellent combination of short response time and reproducible measuring results. When the particles are substantially larger, the response time will become substantially longer, for which reason particles of such a large size are usually not preferred for most common purposes. It is especially of great interest that even with very small particle sizes in the range of 2—5 microns, both a short response time and a splendid reproducibility are obtained.

In order to obtain reproducible results, the particles used according to the present invention should, of course, ideally be monodisperse, i.e. should all by and large be of the same size, which in accordance with the meaning of this term when used in the present context comprises, for example, a particle size distribution of 2—5 microns, whereas a particle size distribution comprising an appreciable part of particles of 2 microns as well as an appreciable part of particles of 15 microns cannot be considered as being monodisperse for the purpose of the present invention.

Preferably, the particles should be approximately spherical or at any rate have essentially equal dimensions in all directions, as particles having a considerably larger dimension in one direction could give rise to an uncertainty on the distance between the membrane and the measuring electrode, depending upon whether the longest dimension of the particles is perpendicular to or parallel with the membrane and the electrode surface. If the particles are very elongated, there is, furthermore, a risk that they may perforate the membrane.

The particles used according to the present invention should to a great extent be chemically inert in relation to the materials with which they are in contact and in relation to the processes which take place during the use of the electrode. Examples of such suitable materials are polymer materials such as nylon and mineral materials such as glass, for example in the form of glass spheres, particles of abrasives which are sharp-edged, for example carborundum particles.

The invention further provides a process for preparing an electrode device for electrochemical measurement of the partial pressure of a gas in a liquid or a gas mixture, said electrode device comprising a measuring surface constituted by or comprising the sensitive part of a measuring electrode and a membrane which is placed in front of the measuring surface and which limits a volume of an electrolyte solution which is in contact with the measuring electrode, said membrane being permeable to the gas to be determined, characterized by applying a dispersion of particles in the electrolyte solution which is to be used in the electrode device to the measuring surface and/or the membrane in an excess, and mounting the membrane on the electrode device in such a manner that excess dispersion is pressed out so that the particles are substantially in a monolayer and make contact with both the measuring surface and the membrane thereby to keep the membrane at a distance from the measuring surface, the particles having such small compressibility that they do not undergo any substantial change of dimensions at pressures occurring during storage, application and handling of the electrode device.

Preferably, the dispersion has a gel-like consistency so that it is stable and easy to apply, which may be obtained in that the dispersion comprises a gelling agent. Suitable gelling agents are such which do not essentially influence the desired function of the electrolyte, e.g. carboxymethylcellulose, which, for example, may suitably be used in a concentration of 3 percent by weight, calculated on the electrolyte solution, or agarose, which may suitably be used in a concentration of about 0.4 percent by weight, calculated on the electrolyte solution. Care should be taken that the gelled dispersion does not contain air bubbles, as these will only escape slowly when mounting the membrane, and, in case they are present in the electrolyte layer of the final electrode, will incur as a consequence that the electrode will not become stable and that the electrolyte volume is not well-defined.

The volume concentration of the particles which, when mounting the membrane, secures a suitable amount of particles for obtaining a monolayer of particles between the membrane and the measuring electrode can easily be ascertained by preliminary practical experiments. Thus, experiments have shown that it is usually sufficient when about 25% of the total area of the measuring surface is covered by particles, as the membrane is thereby kept stretched over the electrode. When carborundum is used as the particles, a coverage of about 25% of the total area of the measuring surface may be obtained by using a dispersion containing about 3 percent by weight of particles having a size of about 3 microns or about 5 percent by weight of particles having a size of about 10 microns. When particles or spheres of a lighter material are used, these normative amounts by weight should, of course, be reduced in a proportion corresponding to the relative proportion between the density of the particles, as, such as stated above, it is the volume used which is decisive to whether the particles are present in a sufficient amount to provide the desired separation between the membrane and the measuring surface. It will be appreciated that a larger volume proportion of the particles might be used, but in principle, it should be aimed at not to use a greater volume concentration of particles than necessary to obtain the necessary well-defined physical spacing between the membrane and the measuring surface, as a larger volume concentration of particles than necessary will accordingly reduce the possible electrolyte volume which may be present.

Suitable materials for use as membrane in the electrode device according to the present invention are, in principle, the same as are suitable for corresponding electrode devices using conventional spacers, for example polyethylene, polypropylene, polytetrafluoro ethylene, fluorinated ethylene propylene, polyethylene terephthalate, polyvinylidene chloride and silicone rubber. The membrane should have such a strength that it is not punctured by the particles used and that it resists the mounting operation during which excess dispersion is pressed out from the interspace between the measuring surface and the membrane. The thickness of the membrane will usually be in the range from about 10 to about 20 microns.

Electrode devices according to the present invention for measuring the partial pressure of carbondioxide typically comprise, for example, a membrane of polytetrafluoroethylene having a thickness of about 12 microns and a bicarbonate solution as electrolyte; electrode devices according to the present invention for measuring the partial pressure of oxygen typically comprise, for example, a membrane of polypropylene having a thickness of about 20 microns and a phosphate solution as electrolyte; and electrode devices according to the present invention for measuring the partial pressure of ammonia typically comprise, for example, a microporous polypropylene membrane having a thickness of about 20 microns and an ammonium chloride solution as electrolyte. Apart from the particles characteristic to the present invention, the electrode devices may be constructed in the same manner as corresponding known electrode devices using conventional spacers, and they may be adapted to their special application purpose in the same manner as the corresponding known electrode devices; they may, especially, be adapted for measuring a liquid sample, for example blood, or they may be adapted for the so-called transcutaneous or non-invasive measurement of the partial pressure of a gas in the blood. In the same manner as the corresponding known electrode devices, the electrode devices according to the present invention are used in connection with a reference electrode such as a saturated calomel electrode or a silver/silver chloride electrode which optionally may be built together with the measuring electrode into a unit in a known manner.

The utilization of the principle of the present invention will not only result in a well-defined small distance between the membrane and the measuring electrode and thereby a well-defined electrolyte layer, but will also ensure that the application of the particle system on the measuring surface is much easier than the application of known spacers, as it is very simple to apply a drop of the particle-containing liquid suspension when the electrode device is placed with the measuring surface upwards, and the particles in the dispersion distribute themselves when the membrane is thereafter quite simply stretched down over the electrode and fastened with suitable straining devices, for example an O-ring, whereas it is associated with not insignificant difficulties to apply a spacer of the known kind upon a drop of liquid on the measuring electrode and, thereafter, to fasten the membrane, as, firstly, one has to work with small discrete pieces of spacer which flow on the drop and, hence, are difficult to keep in place, and, secondly, the separate application of the necessary electrolyte and the spacer requires two operations when using the known technique.

The present invention also provides a dispersion of particles for use in keeping a membrane at a distance from a measuring surface in the electrode device of the invention or in the process of the invention, characterized in that it is a dispersion of particles which have a particle size in the range of 2 to 50 microns in an electrolyte solution which, with respect to its qualitative and quantitative composition, is suitable for use as an electrolyte in a membrane-covered electrode device for polarographic or potentiometric determination of the partial pressure of a gas in a liquid or a gas mixture, the particles having such small compressibility that they do not undergo any substantial change of dimensions at pressures occurring during storage, application and handling of the electrode device. The electrolyte solutions suitable for this purpose typically comprise, inter alia, a pH buffer (compare the above-mentioned solutions), and examples of such electrolyte solutions are the above-mentioned solutions. The particles are preferably particles having a size in the range from 2 to 30 microns, especially in the range from 2 to 20 microns, and are preferably present in the dispersion in a concentration which will lead to the desired concentration of particles in the final monolayer, compare the above explanation. Preferably, a gelling agent, for example of the above-mentioned kind, has been added to the dispersion in order to obtain a viscous consistency.

Norwegian published specification No. 140,028 describes a process for the determination of properties of a metal object by measuring the potential difference between this object and another metal object, in which process an electrolyte paste, for example comprising copper chloride as electrolyte, and additionally comprising paste-forming constituents together with relatively large and non-compressible powder particles of insulating material, is applied to at least one surface of one of the objects, whereafter one surface of the other object, which, for example, in determining the properties of the objects of brass alloys may be a fresh-polished sample of brass, is brought into contact with the paste on the surface of the first object, thus connecting the two objects electrically through the electrolyte paste, whereafter the potential difference between the two objects is measured in a conventional manner by means of a voltmeter, the powder particles serving to keep the objects apart from each other. The particle paste used in this case comprises, such as mentioned above, "relatively large" particles, and it is especially preferred that these are abrasive, as one then quite simply may rub the metal objects against each other to obtain a good contact between metal and electrolyte and to obtain an abrasive effect. This use of relatively large particles in a paste serves the purpose of enabling the factory worker to carry out the measurement in question without difficulties and without the use of laboratory equipment, and this coarse industrial measurement and the materials used for performing the measurement do not render it obvious to use the particle systems in accordance with the present invention for stretching the thin membrane over the measuring surface in electrochemical potentiometric or polarographic electrode devices for measuring the partial pressure of a gas in a liquid, with the obtainment of the new possibilities explained above for these special electrode devices with respect to very thin, well-defined electrolyte layers and the special advantages thereof with respect to response time and with respect to the special easy mounting of the membrane on the electrode device.

The invention will be explained in greater detail in the following, with reference to the drawing, wherein

Figure 1 shows an embodiment of a transcutaneous $Pco_2$-electrode device according to the present invention after the application of the dispersion, but before the mounting of the membrane,

Figure 2 shows the electrode device of Figure 1, in which the membrane has been mounted, and where the particles are in a monolayer,

Figure 3 shows an embodiment of an electrode device according to the present invention for use in in-vitro measurement of $Po_2$ in a blood gas measuring equipment,

Figure 4 shows an embodiment of an electrode device according to the present invention for in-vitro measurement of $Pco_2$ in a blood gas measuring equipment, and

Figure 5 shows a graphical representation of an electrode potential/time-curve for an electrode device according to the present invention.

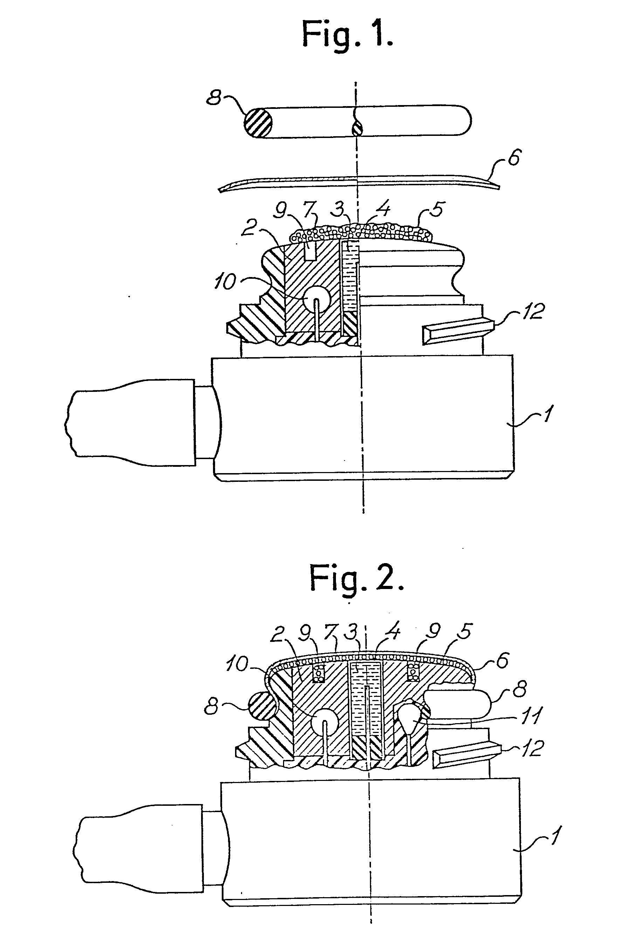

Figures 1 and 2 show an embodiment of an electrode device according to the present invention for transcutaneous $Pco_2$-determination. A reference electrode 2 in the form of a hollow cylindrical body of silver is placed in a housing 1 of an electrically insulating material, for example ABS (acrylonitrile-butadiene-styrene). In the cylindrical cavity of this body, a measuring electrode 3 is positioned which is a pH-measuring electrode, the surface 4 of which is in contact with a sodium bicarbonate solution 5 which serves as electrolyte and is positioned in an electrolyte space which is outwardly delimited by a Teflon® membrane 6. Between the membrane and the surface of the pH-measuring electrode, particles 7 are arranged as a monolayer in the electrolyte and contact both the membrane and the surface of the pH-measuring electrode. The membrane 6 is fixed by means of an O-ring 8. A groove 9 in the silver body serves as an electrolyte reservoir. A zener diode 10 serving as electrical heating means is mounted in the silver body, and a thermistor 11 is mounted as a temperature sensor.

The housing 1 is closed at the back by a lid, and its interior cavity is filled with epoxy resin. The housing 1 may be equipped with a thread 12 for mounting a jacket (not shown) provided with a contact surface which may be supplied with double face adhesive film for applying the electrode against the skin and for eliminating contact between the atmosphere and the carbon dioxide-permeable membrane.

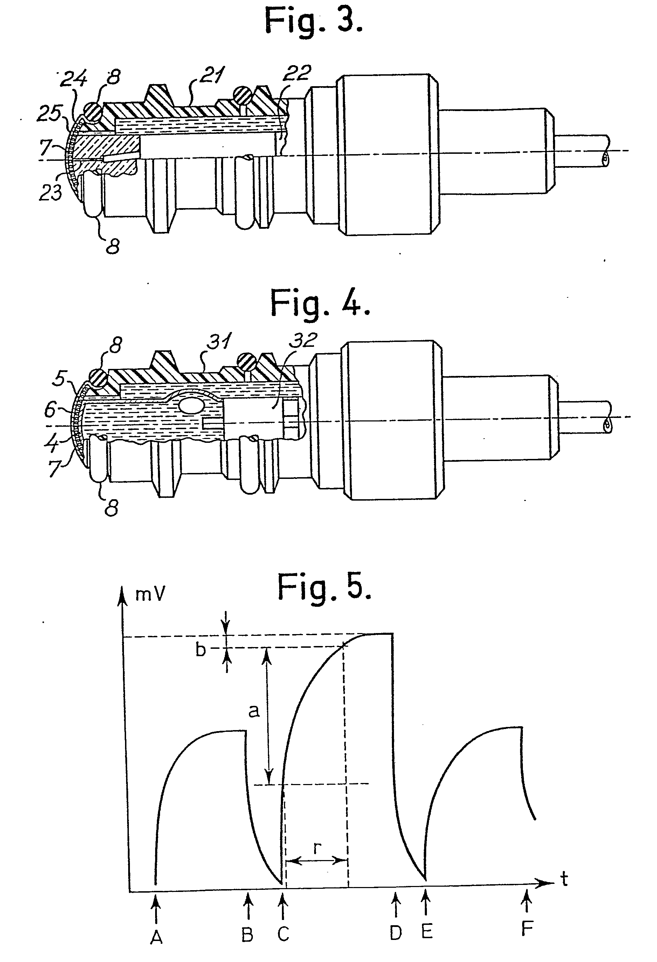

Figure 3 shows an electrode device according to the present invention for measuring $Po_2$ in a self-calibrating blood gas measuring equipment of the type Radiometer ABL. A silver anode 22 in the form of a band at the inner electrode tube is mounted in a housing 21 together with a platinum cathode 23, the measuring surface of which is in contact with a phosphate electrolyte 24, which is in an electrolyte space outwardly delimited by a polypropylene membrane 25. Between the membrane and the surface of the measuring electrode is a monolayer of particles 7. The membrane 25 is fixed by means of an O-ring 8.

Figure 4 shows an electrode device according to the present invention for measuring $Pco_2$ in an ABL blood gas measuring equipment. A silver/silver chloride reference electrode in the form of a band 32 is placed in a plastic housing 31. In the housing, a pH-sensitive glass electrode is mounted having a sensitive surface 4 which is in contact with a sodium bicarbonate solution 5 serving as an electrolyte and being present in an electrolyte space which is outwardly delimited by a Teflon® membrane 6. Between the membrane and the surface of the pH-measuring electrode is a monolayer of particles 7. The membrane 6 is fixed by means of an O-ring 8.

Figure 5 shows a section of the electrode potential/time-curve which was plotted in the experiment of the below Example 5. At A, the electrode is brought into contact with water aerated with a gas mixture containing 4% carbon dioxide; at B, leaching out with water (0% carbon dioxide) begins; at C, the electrode is brought into contact with water aerated with a gas mixture containing 8% carbon dioxide; at D, leaching out with water (0% carbon dioxide) is started; at E, the electrode is brought into contact with water aerated with a gas mixture containing 4% carbon dioxide, and at F, leaching out with water (0% carbon dioxide) begins. r shows the 98% response time, this being the time which passes from the electrode is placed in the liquid in question until 98% of the maximum electrode potential (b/a=2/98) is obtained.

The invention is explained in detail in the following Examples:

Example 1

Preparation of an electrolyte dispersion containing carborundum particles.

An electrolyte is prepared from 12 g of stock electrolyte (S4054-electrolyte for $Pco_2$-electrode, available from Radiometer A/S, Copenhagen, Denmark), said stock electrolyte comprising 1.17 g/kg of sodium chloride, 0.42 g/kg of sodium hydrogen carbonate together with silver chloride and thymol, 0.05 g of agarose and 0.372 g of carborundum 1200 having a particle size of 2—5 microns being added. The carborundum is added to the electrolyte with stirring with a magnetic stirrer, whereafter the agarose is added under stirring. Any air bubbles in the electrolyte dispersion prepared are then removed by evacuating the electrolyte in a desiccator at 10 mm Hg for 30 minutes.

Example 2

An electrolyte dispersion is prepared in the same manner as described in Example 1 from 12 g of stock electrolyte, 0.05 g of agarose and 0.377 g of carborundum 800 having a particle size of about 15 microns.

Example 3

An electrolyte dispersion is prepared in the manner described in Example 1 from 12 g of stock electrolyte, 0.05 g of agarose and 0.381 g of carborundum 400 having a particle size of 30—40 microns.

Example 4

The experiments of Examples 1—3 are repeated, but with 0.36 g of carboxymethyl cellulose instead of 0.05 g of agarose for gelling 12 g of electrolyte.

Example 5

The electrolyte dispersions prepared in Examples 1—3 are incorporated between the measuring electrode and the gas permeable membrane in an electrode device for determining $Pco_2$ in an ABL blood gas measuring equipment, whereby a monolayer of the carborundum particles is obtained, and measurements are carried out using the electrodes mounted in a measuring chamber in the ABL instrument. For comparison, a corresponding electrode device equipped with a nylon spacer having a thickness of 50 microns is used.

Each experiment is carried out as follows:

Firstly, the electrode is rinsed with water, whereafter it is brought into contact with a medium having a known higher $Pco_2$-level (water aerated with a gas mixture containing 4% and 8% carbon dioxide, respectively), and the equilibrium waited for.

On a plotted diagram of the potential as a function of time, a measurement is made of the time for obtaining 98% of the maximum electrode potential obtained at change between two levels which is a generally used parameter for characterizing electrodes. In Figure 5, part of the electrode potential/time-curve of the experiment is sketched from which it appears how the changes between the different $Pco_2$-levels in the medium are made, and how the time for obtaining 98% of the maximum electrode potential change is calculated.

The results obtained appear from the below table where the time in seconds for obtaining 98% of the maximum electrode potential change is listed.

## 0 039 136

TABLE

| | Medium | Spacer | | | |
|---|---|---|---|---|---|
| | | Carbo-rundum 1200 | Carbo-rundum 800 | Carbo-rundum 400 | Nylon |
| Electrode 1 | Water aerated with 8% $CO_2$ | 20 20 21 | 19 21 24 | 29 27 30 | 39 39 |
| | Average response time (sec.) | 20 | 21 | 29 | 39 |
| | Water aerated with 4% $CO_2$ | 24 27 27 | 22 23 24 | 32 39 27 | 45 39 42 |
| | Average response time (sec.) | 26 | 23 | 33 | 42 |
| Electrode 2 | Water aerated with 8% $CO_2$ | 24 27 24 | 24 27 26 | 27 29 27 | 46 51 50 |
| | Average response time (sec.) | 25 | 26 | 28 | 49 |
| | Water aerated with 4% $CO_2$ | 27 27 27 | 32 32 29 | 39 32 28 | 54 53 50 |
| | Average response time (sec.) | 27 | 31 | 33 | 52 |

For each type of spacer, the determination is carried out using two electrodes which are termed electrode 1 and electrode 2.

From the above results, it appears that the response time is substantially reduced when the thickness of the spacer is reduced from about 50 microns (nylon) to about 30—40 microns (carborundum 400). Furthermore, it appears that the equilibration proceeds much faster than particles having a size of the order of 5—15 microns (carborundum 1200 or carborundum 800) are used, and further, it appears that this quick equilibration is not obtained at the expense of the reproducibility, as the individual measuring results (3 measurements per electrode per carbon dioxide level) do not deviate substantially from each other.

**Claims**

1. An electrode device (1) for electrochemical measurement of the concentration or the partial pressure of a gas in a liquid or a gas mixture, comprising a measuring surface (4) constituted by or comprising the sensitive part of a measuring electrode (3) and a membrane (6) which is placed in front of the measuring surface and limits a volume of an electrolyte solution (5) which is in contact with the measuring electrode (3), said membrane being permeable to the gas species to be determined, characterized in that the electrode device comprises particles (7) dispersed substantially in a monolayer in the electrolyte solution between the measuring surface (4) and the membrane (6) in a sufficient amount to keep the membrane in a distance from the measuring surface (4), the monolayer making contact with both the measuring surface (4) and the membrane (6), the particles (7) having such small compressibility that they do not undergo any substantial change of dimensions at pressure occurring during storage, application and handling of the electrode device.

2. An electrode device according to claim 1, wherein the particles (7) have an approximately spherical shape.

3. An electrode device according to claim 2, wherein the particles (7) are particles of abrasives or spheres of polymer or glass.

7

4. An electrode device according to claim 3, wherein the particles (7) are particles of carborundum.

5. An electrode device according to any one of claims 1 to 4, wherein the particles (7) have a size in the range of 2 to 50 microns.

6. An electrode device according to claim 5, wherein the particles have a size in the range of 2 to 30 microns.

7. An electrode device according to claim 6, wherein the particles have a size in the range of 2 to 20 microns.

8. An electrode device according to any one of claims 5 to 7, wherein the particles (7) are substantially of the same size.

9. An electrode device according to any preceding claim, wherein the thickness of the membrane is in the range of 12 to 30 microns.

10. An electrode device according to any preceding claim, wherein the measuring electrode (3) is a glass electrode or a platinum electrode.

11. A process for preparing an electrode device (1) for electrochemical measurement of the partial pressure of a gas in a liquid or a gas mixture, said electrode device comprising a measuring surface (4) constituted by or comprising the sensitive part of a measuring electrode (3) and a membrane (6) which is placed in front of the measuring surface (4) and which limits a volume of an electrolyte solution (5) which is in contact with the measuring electrode (3), said membrane being permeable to the gas to be determined, characterized by applying a dispersion of particles (7) in the electrolyte solution (5) which is to be used in the electrode device (1) to the measuring surface (4) and/or the membrane (6) in an excess, and mounting the membrane (6) on the electrode device in such a manner that excess dispersion is pressed out so that the particles (7) are substantially in a monolayer and make contact with both the measuring surface (4) and the membrane (6) thereby to keep the membrane (6) at a distance from the measuring surface (4), the particles (7) having such small compressibility that they do not undergo any substantial change of dimensions at pressures occurring during storage, application and handling of the electrode device.

12. A process according to claim 11, wherein the dispersion is gelled to such an extent that it is stable.

13. A process according to claim 12, wherein the dispersion is gelled using a thickening agent.

14. A process according to claim 13, wherein the thickening agent is carboxymethyl cellulose.

15. A process according to claim 14, wherein the carboxymethyl cellulose is present in an amount of about 3%.

16. A process according to claim 13, wherein the thickening agent is agarose.

17. A process according to claim 16, wherein the agarose is present in an amount of about 0.4%.

18. A dispersion of particles for use in keeping a membrane (6) at a distance from a measuring surface (4) in the electrode device of any one of claims 1 to 10 or in the process of any one of claims 11 to 17, characterized in that it is a dispersion of particles which have a particle size in the range of 2 to 50 microns and a size distribution of less than 13 microns in an electrolyte solution which, with respect to its qualitative and quantitative composition, is suitable for use as an electrolyte in a membrane-covered electrode device for polarographic or potentiometric determination of the partial pressure of a gas in a liquid or a gas mixture, the particles having such small compressibility that they do not undergo any substantial change of dimensions at pressures occurring during storage, application and handling of the electrode device.

19. A dispersion according to claim 18, wherein the electrolyte solution contains a pH-buffer.

20. A dispersion according to claim 18 or 19, wherein the particles are particles of an abrasive, glass or polymer.

21. A dispersion according to claim 20, wherein the particles are of carborundum.

22. A dispersion according to claim 20 or 21, wherein the particles have a size in the range of 2 to 20 microns.

23. A dispersion according to claim 22, comprising carborundum particles having a size of about 3 microns in an amount of about 3 percent by weight or carborundum particles having a size of about 10 microns in an amount of about 5 percent by weight.

**Patentansprüche**

1. Elektrodenvorrichtung (1) zur elektrochemischen Messung der Konzentration oder des Partialdruckes eines Gases in einer Flüssigkeit oder in einer Gasmischung, umfassend eine Meßfläche (4), aufgebaut aus oder umfassend den empfindlichen Teil einer Meßelektrode (3) und eine Membran (6), welche vor der Meßfläche angeordnet ist und ein Volumen einer Elektrolytlösung (5), welche in Kontakt mit der Meßelektrode (3) ist, begrenzt, wobei die Membran gegenüber den zu bestimmenden Gasspezies permeabel ist, dadurch gekennzeichnet, daß die Elektrodenvorrichtung Teilchen (7) umfaßt, welche im wesentlichen in einer einzelnen Schicht in der Elektrolytlösung zwischen der Meßfläche (4) und der Membran (6) in einer ausreichenden Menge dispergiert sind, um die Membran in einer Entfernung von der Meßfläche (4) zu halten, wobei die einzelne Schicht sowohl mit der Meßfläche (4) als auch der Membran (6) in Kontakt ist und die Teilchen (7) eine so kleine Kompressibilität besitzen, daß sie keine wesentliche Dimensionsänderung bei Drucken, welche während der Lagerung, Anwendung und Handhabung der Elektrodenvorrichtung auftreten, erfahren.

2. Elektrodenvorrichtung nach Anspruch 1, worin die Teilchen (7) etwa eine sphärische Form besitzen.

3. Elektrodenvorrichtung nach Anspruch 2, worin die Teilchen (7) Teilchen von Schleifmitteln oder Kugeln eines Polymers oder Glases sind.

4. Elektrodenvorrichtung nach Anspruch 3, worin die Teilchen (7) Karborundteilchen sind.

5. Elektrodenvorrichtung nach einem der Ansprüche 1 bis 4, worin die Teilchen (7) eine Größe im Bereich von 2 bis 50 Mikron besitzen.

6. Elektrodenvorrichtung nach Anspruch 5, worin die Teilchen eine Größe im Bereich von 2 bis 30 Mikron besitzen.

7. Elektrodenvorrichtung nach Anspruch 6, worin die Teilchen eine Größe im Bereich von 2 bis 20 Mikron besitzen.

8. Elektrodenvorrichtung nach einem der Ansprüche 5 bis 7, worin die Teilchen (7) im wesentlichen die gleiche Größe besitzen.

9. Elektrodenvorrichtung nach einem der vorhergehenden Ansprüche, worin die Dicke der Membran im Bereich von 12 bis 30 Mikron liegt.

10. Elektrodenvorrichtung nach einem der vorhergehenden Ansprüche, worin die Meßelektrode (3) eine Glaselektrode oder eine Platinelektrode ist.

11. Verfahren zur Herstellung einer Elektrodenvorrichtung (1) zur elektrochemischen Messung des Partialdrucks eines Gases in einer Flüssigkeit oder einer Gasmischung, wobei die Elektrodenvorrichtung eine Meßfläche (4) umfaßt, aufgebaut aus oder umfassend den empfindlichen Teil einer Meßelektrode (3) und eine Membran (6), welche vor der Meßfläche (4) angeordnet ist und ein Volumen einer Elektrolytlösung (5), welche in Kontakt mit der Meßelektrode (3) ist, begrenzt, wobei die Membran gegenüber den zu bestimmenden Gasspezies permeabel ist, gekennzeichnet durch Aufbringen einer Dispersion von Teilchen (7) in der Elektrolytlösung (5), welche in der Elektrodenvorrichtung (1) verwendet wird, auf die Meßfläche (4) und/oder die Membran (6) im überschuß und Befestigen der Membran (6) auf der Elektrodenvorrichtung auf solch eine Weise, daß die überschüssige Dispersion herausgepreßt wird, so daß die Teilchen (7) im wesentlichen in einer einzelnen Schicht vorliegen und mit der Meßfläche (4) und der Membran (6) in Kontakt sind, um dadurch die Membran (6) in einer Entfernung von der Meßfläche (4) zu halten, wobei die Teilchen (7) eine so kleine Kompressibilität besitzen, daß sie keine wesentliche Dimensionsänderung bei Drucken, die während der Lagerung, Anwendung und Handhabung der Elektrodenvorrichtung auftreten, erfahren.

12. Verfahren nach Anspruch 11, worin die Dispersion auf solch ein Ausmaß geliert wird, daß sie stabil ist.

13. Verfahren nach Anspruch 12, worin die Dispersion unter Verwendung eines Verdickungsmittels geliert wird.

14. Verfahren nach Anspruch 13, worin das Verdickungsmittel Carboxymethylcellulose ist.

15. Verfahren nach Anspruch 14, worin die Carboxymethylcellulose in einer Menge von etwa 3% vorliegt.

16. Verfahren nach Anspruch 13, worin das Verdickungsmittel Agarose ist.

17. Verfahren nach Anspruch 16, worin die Agarose in einer Menge von etwa 0,4% vorliegt.

18. Dispersion von Teilchen zur Verwendung, um eine Membran (6) in einer Entfernung von einer Meßfläche (4) in der Elektrodenvorrichtung nach einem der Ansprüche 1 bis 10 oder in dem Verfahren nach einem der Ansprüche 11 bis 17 zu halten, dadurch gekennzeichnet, daß sie eine Dispersion von Teilchen ist, welche eine Teilchengröße im Bereich von 2 bis 50 Mikron und eine Größenverteilung von weniger als 13 Mikron in einer Elektrolytlösung besitzen, welche in bezug auf ihre qualitative und quantitative Zusammensetzung zur Verwendung als Elektrolyt in einer membranbedeckten Elektrodenvorrichtung für die polarographische oder potentiometrische Bestimmung des Partialdrucks eines Gases in einer Flüssigkeit oder eine Gasmischung geeignet ist, wobei die Teilchen eine so kleine Kompressibilität besitzen, daß sie keine wesentliche Dimensionsänderung bei Drucken, die während der Lagerung, Anwendung und Handhabung der Elektrodenvorrichtung auftreten, erfahren.

19. Dispersion nach Anspruch 18, worin die Elektrolytlösung einen pH-Puffer enthält.

20. Dispersion nach Anspruch 18 oder 19, worin die Teilchen eines Schmiermittels, Glases oder Polymers sind.

21. Dispersion nach Anspruch 20, worin die Teilchen aus Karborund sind.

22. Dispersion nach Anspruch 20 oder 21, worin die Teilchen eine Größe im Bereich von 2 bis 20 Mikron besitzen.

23. Dispersion nach Anspruch 22, umfassend Karborundteilchen mit einer Größe von etwa 3 Mikron in einer Menge von etwa 3 Gew.-% oder Karborundteilchen in einer Größe von etwa 10 Mikron in einer Menge von etwa 5 Gew.-%.

**Revendications**

1. Système d'électrode (1) pour la mesure électrochimique de la concentration ou de la pression partielle d'un gaz dans un liquide ou dans un mélange gazeux, comprenant une surface de mesure (4) constituée par ou comprenant la partie sensible d'une électrode de mesure (3) et une membrane (6) qui est placée en avant de la surface de mesure et qui limite un volume de solution électrolyte (5) qui est en contact avec l'électrode de mesure (3), ladite membrane étant perméable au type de gaz à déterminer, caractérisé en ce que le système d'électrode comprend des particules (7) dispersées sensiblement en une couche à une seule assise dans la solution électrolyte, entre la surface de mesure (4) et la membrane (6), dans une quantité suffisante pour maintenir la membrane espacée de la surface de mesure (4), la couche à une seule assise étant en contact à la fois avec la surface de mesure (4) et avec la membrane (6), les particules (7) ayant une compressibilité tellement faible qu'elles ne subissent aucun changement notable de dimension à la pression qui se manifeste pendant le stockage, l'utilisation et la manipulation du système d'électrode.

2. Système d'électrode selon la revendication 1, dans lequel les particules (7) ont une forme approximativement sphérique.

3. Système d'électrode selon la revendication 2, dans lequel les particules (7) sont des particules d'abrasifs ou des sphères de polymère ou de verre.

4. Système d'électrode selon la revendication 3, dans lequel les particules (7) sont des particules de carborundum.

5. Système d'électrode selon une quelconque des revendications 1 à 4, dans lequel les particules (7) ont une dimension comprise dans l'intervalle allant de 2 à 50 microns.

6. Système d'électrode selon la revendication 5, dans lequel les particules ont une dimension comprise dans l'intervalle allant de 2 à 30 microns.

7. Système d'électrode selon la revendication 6, dans lequel les particules ont une dimension comprise dans l'intervalle allant de 2 à 20 microns.

8. Système d'électrode selon une quelconque des revendications 5 à 7, dans lequel les particules (7) sont sensiblement de la même dimension.

9. Système d'électrode selon une revendication précédente quelconque, dans lequel l'épaisseur de la membrane est comprise dans l'intervalle allant de 12 à 30 microns.

10. Système d'électrode selon une revendication précédente quelconque, dans lequel l'électrode de mesure (3) est une électrode de verre ou une électrode de platine.

11. Procédé de préparation d'un système d'électrode (1) pour la mesure électrochimique de la pression partielle d'un gaz dans un liquide ou dans un mélange gazeux, ce système d'électrode comprenant une surface de mesure (4) constituée par ou comprenant la partie sensible d'une électrode de mesure (3) et une membrane (6) qui est placée en avant de la surface de mesure (4) et qui limite un volume de solution électrolyte (5) qui est en contact avec l'électrode de mesure (3), ladite membrane étant perméable au gaz à déterminer, caractérisé en ce qu'on applique une dispersion de particules (7) dans la solution électrolyte (5) qu'il s'agit d'utiliser dans le système d'électrode (1) sur la surface de mesure (4) et/ou sur la membrane (6), dans une quantité excédentaire, et on monte la membrane (6) sur le système d'électrode de telle manière que l'excès de dispersion soit expulsé, de telle façon que les particules (7) soient sensiblement en une couche à une seule assise et entrent en contact à la fois avec la surface de mesure (4) et avec la membrane (6), pour maintenir ainsi la membrane (6) espacée de la surface de mesure (4), les particules (7) possédant une compressibilité tellement faible qu'elles ne subissent aucun changement notable de dimension aux pressions qui se manifestent pendant le stockage, l'utilisation et la manipulation du système d'électrode.

12. Procédé selon la revendication 11, dans lequel la dispersion est gélifiée à un degré tel qu'elle soit stable.

13. Procédé selon la revendication 12, dans lequel la dispersion est gélifiée au moyen d'un agent épaississant.

14. Procédé selon la revendication 13, dans lequel l'agent épaississant est la carboxyméthyl cellulose.

15. Procédé selon la revendication 14, dans lequel la carboxyméthyl cellulose est présente dans une quantité d'environ 3 pourcent.

16. Procédé selon la revendication 13, dans lequel l'agent épaississant est l'agarose.

17. Procédé selon la revendication 16, dans lequel l'agarose est présente dans une quantité d'environ 0,4 pourcent.

18. Dispersion de particules destinée à être utilisée pour maintenir une membrane (6) espacée d'une surface de mesure (4), dans le système d'électrode selon l'une quelconque des revendications 1 à 10, ou dans le procédé selon l'une quelconque des revendications 11 à 17, caractérisée en ce qu'elle est constituée par une dispersion de particules qui ont une dimension de particules comprise dans l'intervalle allant de 2 à 50 microns, et une distribution de dimensions de moins de 13 microns, dans une solution électrolyte qui, en ce que concerne sa composition qualitative et quantitative, est appropriée pour être utilisée comme électrolyte dans un système d'électrode recouvert d'une membrane destiné à la détermination polarographique ou potentiomètrique de la pression partielle d'un gaz dans un liquide ou dans un mélange gazeux, les particules possédant une compressibilité tellement faible qu'elles ne subissent aucun changement notable de dimension aux pressions qui se manifestent pendant le stockage, l'utilisation et la manipulation du système d'électrode.

19. Dispersion selon la revendication 18, dans laquelle la solution électrolyte contient un tampon de pH.

20. Dispersion selon la revendication 18 ou 19, dans laquelle les particules sont des particules d'un abrasif, de verre ou de polymère.

21. Dispersion selon la revendication 20, dans laquelle les particules sont faites de carborundum.

22. Dispersion selon la revendication 20 ou la revendication 21, dans laquelle les particules ont une dimension comprise dans l'intervalle allant de 2 à 20 microns.

23. Dispersion selon la revendication 22, comprenant des particules de carborundum ayant une dimension d'environ 3 microns, en une quantité d'environ 3 pourcent en poids, ou des particules de carborundum ayant une dimension d'environ 10 microns en une quantité d'environ 5 pourcent en poids.

# Fig. 1.

# Fig. 2.

# Fig. 3.

# Fig. 4.

# Fig. 5.